(19) Europäisches Patentamt

European Patent Office

Office européen des brevets

(11) **EP 0 771 217 B1**

(12) **EUROPÄISCHE PATENTSCHRIFT**

(45) Veröffentlichungstag und Bekanntmachung des Hinweises auf die Patenterteilung:
**07.04.2004 Patentblatt 2004/15**

(21) Anmeldenummer: **95943492.9**

(22) Anmeldetag: **10.07.1995**

(51) Int Cl.[7]: **A61K 47/48**, A61K 31/567

(86) Internationale Anmeldenummer:
**PCT/EP1995/002656**

(87) Internationale Veröffentlichungsnummer:
**WO 1996/002277 (01.02.1996 Gazette 1996/06)**

(54) **FESTE ARZNEIFORMEN, ENTHALTEND CLATHRATE VOM STEROIDALEN SEXUALHORMON ETHINYLESTRADIOL**

SOLID DRUG FORMS CONTAINING CLATHRATES OF THE STEROID SEX HORMONE ETHINYLESTRADIOL

FORMES DE MEDICAMENTS SOLIDES CONTENANT DES CLATHRATES DE L'HORMONE ETHINYLESTRADIOL

(84) Benannte Vertragsstaaten:
**AT BE CH DE DK ES FR GB GR IE IT LI LU MC NL PT SE**

(30) Priorität: **20.07.1994 DE 4426709**

(43) Veröffentlichungstag der Anmeldung:
**07.05.1997 Patentblatt 1997/19**

(73) Patentinhaber: **SCHERING AKTIENGESELLSCHAFT
13342 Berlin (DE)**

(72) Erfinder:
• **BACKENSFELD, Thomas
D-10559 Berlin (DE)**
• **TACK, Johannes
D-13595 Berlin (DE)**

(56) Entgegenhaltungen:
**US-A- 4 596 795          US-A- 4 877 774**

• **DATABASE WPI Section Ch, Week 8828 Derwent Publications Ltd., London, GB; Class A11, AN 88-195856 & JP,A,63 135 402 ( TOKUYAMA SODA KK) , 7.Juni 1988 & CHEMICAL ABSTRACTS, vol. 110, no. 20, 1989 Columbus, Ohio, US; abstract no. 179512, & PATENT ABSTRACTS OF JAPAN vol. 12 no. 391 (C-537) ,18.Oktober 1988**

• **DATABASE WPI Section Ch, Week 8232 Derwent Publications Ltd., London, GB; Class A96, AN 82-66933E & JP,A,57 106 698 ( KYOWA HAKKO KOGYO KK) , 2.Juli 1982 & CHEMICAL ABSTRACTS, vol. 98, no. 2, 1983 Columbus, Ohio, US; abstract no. 8163, & PATENT ABSTRACTS OF JAPAN vol. 6 no. 197 (C-128) ,6.Oktober 1982**

• **INT. J. PHARM., 1982, VOL. 10, NO. 1, PAGES 1-15, UEKAMA, K. ET AL 'Inclusion complexations of steroid hormones with cyclodextrins in water and in solid phase'**

• **PHARMAZIE, 1989, VOL. 44, NO. 9, PAGES 623-5, KRALOVA, K. ET AL 'Interactions of.beta.-cyclodextrin with steroid compounds in aqueous solutions'**

• **BIOCHIM. BIOPHYS. ACTA, 1987, VOL. 923, NO. 1, PAGES 83-7, KEMPFLE, M. A. ET AL 'The binding of fluorescent 4,6,8(14)-triene-3-one steroids to cyclodextrins as a model for steroid-protein interactions'**

• **PHARM. WEEKBL. SCI. ED., 1992, VOL. 14, NO. 4 A, PAGE(S) 253-257, NETHERLANDS, HERMENS W.A.J.J. 'Delivery of hormones: Some new concepts'**

• **TOPICS IN PHARMACEUTICAL SCIENCES, EDS. D.D. BREIMER AND P. SPEISER, 1987, ELSEVIER SCIENCE PUBLISHERS B.V. (BIOMEDICAL DIVISION), PAGES 181-194, K. UEKAMA 'CYCLODEXTRIN INCLUSION COMPOUNDS: EFFECTS ON STABILITY AND BIO-PHARMACEUTICAL PROPERTIES'**

EP 0 771 217 B1

Bemerkungen:
    Die Akte enthält technische Angaben, die nach dem
Eingang der Anmeldung eingereicht wurden und die
nicht in dieser Patentschrift enthalten sind.

Bemerkungen:
    Die Akte enthält technische Angaben, die nach dem
Eingang der Anmeldung eingereicht wurden und die
nicht in dieser Patentschrift enthalten sind.

**Beschreibung**

Die Erfindung betrifft die Verwendung von β-Cyclodextrin in einer Kombination enthaltend 17α-Ethinytestradiol.

Bekanntlich sind natürliche und insbesondere auch synthetisch abgeleitete Sexualhormone in der Regel hochwirksame Arzneimittelwirkstoffe. Demzufolge enthalten feste Arzneiformen diese Wirkstoffe meist in sehr geringer Dosierung, dies sind üblicherweise 0,01 μg bis 500 μg und insbesondere 0,1 μg bis 200 μg pro einzeldosierte Arzneiform. Dies hat zur Folge, dass sowohl die Zubereitung als auch die Lagerung und die Anwendung dieser Arzneiformen oft problematisch ist.

Bei der Zubereitung derart gering dosierter Arzneiformen treten fast unvermeidlich starke Schwankungen der Wirkstoffkonzentrationen in den Dosiseinheiten auf (mangelnde content uniformity), die um so stärker in Erscheinung treten, je geringer der Wirkstoff dosiert ist.

Bei der Lagerung derartig gering dosierter Präparate beobachtet man zudem oft zusätzlich noch eine Abnahme der Wirkstoffkonzentration als Folge von meist oxidativen Abbaureaktionen des Wirkstoffs.

Hinzu kommt, dass die Bioverfügbarkeit des Wirkstoffes in derart geringer Dosierung einem ausgeprägten first pass Effekt unterliegt und große inter- und intraindividuelle Schwankungen aufweist.

Uekama, K. et. al (Int. J. Pharm., 1982, Vol. 10, No. 1, S. 1-15 "Inclusion complexations of steroid hormones with cyclodextrins in water and in solid phase") untersucht β- und γ-Cyclodextrine in Kombination mit mehreren Steroiden, nicht jedoch mit 17α-Ethinylestradiol.

US-A-4,877,774 befaßt sich mit einer Steigerung der Absorption von Steroidhormonen durch den Einsatz von γ-Cyclodextrin zur besseren Durchdringung der Schleimhäute. Es wird aber beschrieben dass β-Cyclodextrin sich als unbrauchbar für die Absorption von Medikamenten herausgestellt hat.

US-A-4,596,795 beschreibt die Verwendung von Poly-β-Cyclodextrin und Hydroxy-propyl-β-Cyclodextrin zur Steigerung der Absorption von Testosteron, Progesteron und Estradiol.

JP-A-57106698 beschreibt ein Medroxyprogesteronacetat in β-Cyclodextrin. Dadurch wird die Wasserlöslichkeit der Steroide erhöht, wodurch die Resorption durch den Verdauungstrakt verbessert wird.

In JP-A-860220683 wird die Verwendung von unter anderem Progesteron und Testosteron offenbart, welches mit Maltosyl-β-Cyclodextran kombiniert wird, um die Löslichkeit des Steroids zu steigern.

Hermens W. A. J. J. (Pharm. Weekbl. Sci. Ed., 1992, Vol. 14, No. 4a, S. 253-257 "Delivery of hormones: Some new concepts") beschreibt einen Vaginalring zur Kontrazeption, welcher 3-Ketodesogestrel und 17α-Ethinylestradiol enthält. Das darin genannte Dimethyl-β-Cyclodextrin wird als Enhancer für die nasale Absorption von Estradiol eingesetzt.

Uekema K., (Topics in Pharmaceutical Sciences, Ed. D. D. Breimer und P. Speiser, 1987, Elsevier Science Publishers B. V. (Biomedical Divison), S. 181-194 "Cyclodextrin inclusion compounds: Effects on stability and biopharmaceutical properties") beschreibt die Verwendung von α-, β- und γ-Cyclodextrin und deren Derivaten zur Steigerung der Stabilität und der Bioverfügbarkeit von Prostaglandinen, Prostacyclin, Chlorpromazin, Carmofur und Nitraten. Dabei wurden individuelle Kombinationen aus Substanzen und aus einem der Cyclodextrinen hergestellt.

Es wurde nun gefunden, dass man die Nachteile, welche man insbesondere bei der Zubereitung und Lagerung von Arzneiformen, die niedrig dosierte steroidale Sexualhormone enthalten, beobachtet, zumindest weitgehend vermeiden kann, wenn man Arzneiformen bereitstellt, die pulverförmige Cyclodextrin-Clathrate dieser Wirkstoffe enthalten.

Aufgabe der vorliegenden Erfindung ist es, 17α-Ethinylestradiol eine bessere Lagerungsfähigkeit in festen Arzneiformen zu geben.

Die Aufgabe wird durch die Verwendung von β-Cyclodextrin in einer Kombination enthaltend 17α-Ethinylestradiol gelöst, wobei die Kombination in einer festen Arzneiform vorliegt und β-Cyclodextrin zur Reduktion der Abnahme des Wirkstoffes verwendet wird.

Erfindungsgemäß ist die Verwendung, wobei die Abnahme des Wirkstoffs durch oxidative Abbaureaktionen bedingt ist.

Die erfindungsgemäße Verwendung ist dadurch gekennzeichnet, dass das 17α-Ethinylestradiol eine Einzeldosierung von 0,01 μg bis 200 μg aufweist.

Die erfindungsgemäße Verwendung ist weiterhin dadurch gekennzeichnet, dass das 17α-Ethinylestradiol eine Einzeldosierung von 0,1 μg bis 200 μg aufweist.

Die erfindungsgemäße Verwendung ist schließlich dadurch gekennzeichnet, dass das 17α-Ethinylestradiol eine Einzeldosierung von 10 oder 20 μg aufweist.

Derartige Cyclodextrine sind vorzugsweise das das Dimethyl-β-cyclodextrin, das 2-Hydroxyethyl-β-cyclodextrin, das 2-Hydroxypropyl-β-cyclodextrin und insbesondere das β-Cyclodextrin (Drug Dev. and Ind. Pharm., 17, 1991, 1503-1549, J. Incl. Phenom., 1, 1983, 135-150 und die WO 93/13138). Zur Herstellung der Clathrate kann man das Steroidhormon 17α-Ethinylestradiol mit dem Cyclodextrin gegebenenfalls unter Zusatz weiterer pharmazeutischer Hilfsstoffe innigst mischen (beispielsweise durch Rühren, Kneten) oder man kann aus einer Lösung der Komponenten in Wasser und/oder einem geeigneten Lösungsmittel (wie zum Beispiel einem $C_1$-$C_4$-Alkohol wie Methanol, Ethanol oder Isopropanol oder einem $C_2$-$C_4$ Keton wie Aceton oder Methylethylketon) das Lösungsmittel beispielsweise durch Vakuumdestillation, Gefriertrocknung oder Sprühtrocknung entfernen. Andererseits ist es aber auch möglich, das in einem geeigneten Lösungsmittel (wie zum Beispiel einem der oben genannten Alkohole oder Keton) gelöste Steroidhormon in eine wässrige Cyclodextrin-Lösung einzutragen, das ausgefallene Clathrat abzufiltrieren und zu trocknen.

In gleicher Weise wie die Wirkstoffe selbst können dann auch die Clathrate gegebenenfalls nach Zugabe der üblichen Hilfsmittel wie zum Beispiel Lactose, Stärke, Polyvinylpyrrolidon, Magnesiumstearat und Konservierungsmittel in die gewünschten Arzneiformen, wie Tabletten, Pulver, Granulate etc. verarbeitet werden.

Es ist für den Fachmann offenkundig, dass es einiger geläufiger Vorversuche bedarf, um zu ermitteln, welches Cyclodextrin optimalerweise zum Einschluss des gewünschten steroidalen Steroidhormons geeignet ist. Normalerweise wählt man das Verhältnis von Cyclodextrin zu Steroidhormon so, dass 1: 1 mol: mol Komplexe gebildet werden, was aber nicht ausschließt, das es im Einzelfall günstiger ist, das molare Verhältnis so zu wählen, dass beispielsweise 2:1, 3:1, 3:2 oder 1:2 Komplexe gebildet werden.

Die nachfolgenden Ausführungsbeispiele dienen zur näheren Erläuterung der Erfindung:

**Beispiel 1**

20,96 g 17$\alpha$-Ethinylestradiol werden in 20 ml Ethanol gelöst. 28,38 g $\beta$-Cyclodextrin (berechnet auf wasserfreies $\beta$-Cyclodextrin) werden unter Rühren bei 45 ° C in 900 ml Wasser gelöst. Die ethanolische Ethinylestradiollösung wird innerhalb von 40 Minuten unter Rühren so zur wässrigen Cyclodextrinlösung zugetropft, dass eine leicht getrübte Lösung entsteht. Innerhalb von 2 Stunden wird die Lösung auf 25 ° C abgekühlt. Es wird weitere 20 Stunden bei 25 ° C gerührt. Der ausgefallene Feststoff wird abgesaugt und zweimal mit je 50 ml Wasser gewaschen. Das Kristallisat wird zweimal mit je 40 ml Aceton aufgeschlämmt und abgesaugt. Anschließend wird mit 50 ml Wasser nachgewaschen. Das feuchte Kristallisat wird im Vakuum über Phosphorpentoxid getrocknet.

Der Gehalt an 17$\alpha$-Ethinylestradiol in der Einschlussbindung wird mittels Hochdruckflüssigkeitschromatographie ermittelt und beträgt 10,2%.

**Beispiel 2**

2,37 g $\beta$-Cyclodextrin werden in 200 ml Wasser gelöst. Zu der wässrigen Cyclodextrinlösung werden 118,6 mg 17$\alpha$-Ethinylestradiol eingewogen. Die Suspension wird für 48 Stunden gerührt. Der Feststoff wird abgesaugt und zweimal mit je 25 ml Wasser gewaschen. Das Kristallisat wird zweimal mit je 20 ml Aceton aufgeschlämmt und abgesaugt. Anschließend wird mit 20 ml Wasser nachgewaschen. Das feuchte Kristallisat wird im Vakuum über Phosphorpentoxid getrocknet.

Der Gehalt an Ethinylestradiol in der Einschlussbindung wird mittels Hochdruckflüssigkeitschromatographie ermittelt und beträgt 10,4%.

**Beispiel 3**

Ein $\beta$-Cyclodextrineinschlusskomplex (hergestellt nach Beispiel 1) wird gemahlen und portionsweise mit Lactose gerieben. Es wird Maisstärke und modifizierte Stärke zugemischt. Mit einer wässrigen Polyvinylpyrrolidon 25000 Lösung wird das Pulver im Wirbelschichtgranulator zu einem Granulat verarbeitet. Nach dem Untermischen von Magnesiumstearat wird die erhaltene Pressmasse zu Tabletten mit einem Gewicht von 55 mg und einem Durchmesser von 5 mm verpresst.

| Zusammensetzung einer Tablette: | |
| --- | --- |
| Ethinylestradiol/$\beta$-Cyclodextrin | 0,098 mg |
| Einschlussverbindung = 10 µg 17$\alpha$-Ethinylestradiol | |
| Lactose | 35,102 mg |
| Maisstärke | 9,900 mg |
| Modifizierte Stärke | 6,600 mg |
| Polyvinylpyrrolidon 25000 | 2,750 mg |
| Magnesiumstearat | 0,550 mg |
| | 55,000 mg |

**EP 0 771 217 B1**

**Beispiel 4**

Ein β-Cyclodextrineinschlußkomplex (hergestellt nach Beispiel 2) wird gemahlen. 9,615 g des Komplexes (entsprechend 1 g Ethinylestradiol) werden in einer Pulvermischung aus 2360,385 g Lactose, 1300 g mikrokristalliner Cellulose und 310 mg Maisstärke homogen eingearbeitet. Nach Zugabe von 20 g Magnesiumstearat wird die erhaltene Pulverpressmasse mit einer Tablettenpresse zu Tabletten mit 6 mm Durchmesser und einem Tablettengewicht von 80 mg verpresst. Sie verfügten über einen Wirkstoffgehalt von 20 µg Ethinylestradiol pro Tablette.

**Patentansprüche**

1. Verwendung von β-Cyclodextrin in einer Kombination enthaltend 17α-Ethinylestradiol,
   **dadurch gekennzeichnet,**
   **dass** die Kombination in einer festen Arzneiform vorliegt und
   **dass** β-Cyclodextrin zur Reduktion der Abnahme des Wirkstoffes verwendet wird.

2. Verwendung nach Anspruch 1, **dadurch gekennzeichnet, dass** die Abnahme des Wirkstoffs durch oxidative Abbaureaktionen bedingt ist.

3. Verwendung nach Anspruch 1 oder 2, **dadurch gekennzeichnet, dass** das 17α-Ethinylestradiol eine Einzeldosierung von 0,01 µg bis 200 µg aufweist.

4. Verwendung nach Anspruch 3, **dadurch gekennzeichnet, dass** das 17α-Ethinylestradiol eine Einzeldosierung von 0,1 µg bis 200 µg aufweist.

5. Verwendung nach Anspruch 4, **dadurch gekennzeichnet, dass** das 17α-Ethinylestradiol eine Einzeldosierung von 10 oder 20 µg aufweist.

**Claims**

1. Use of β-cyclodextrin in a combination containing 17α-ethinylestradiol,
   **characterized in that**
   the combination is present in a solid dosage form and **in that** β-cyclodextrin is used to reduce the decrease in the active ingredient.

2. Use according to Claim 1, **characterized in that** the decrease in the active ingredient is due to oxidative degradation reactions.

3. Use according to Claim 1 or 2, **characterized in that** 17α-ethinylestradiol has a single dose of 0.01 µg to 200 µg.

4. Use according to Claim 3, **characterized in that** 17α-ethinylestradiol has a single dose of 0.1 µg to 200 µg.

5. Use according to Claim 4, **characterized in that** 17α-ethinylestradiol has a single dose of 10 or 20 µg.

**Revendications**

1. Utilisation de la β-cyclodextrine dans une combinaison comprenant du 17α-éthinylestradiol, **caractérisée en ce que** la combinaison se trouve sous une forme galénique solide et **en ce que** la β-cyclodextrine est utilisée pour réduire la diminution de l'ingrédient actif.

2. Utilisation selon la revendication 1, **caractérisée en ce que** la diminution de l'ingrédient actif est due à des réactions de dégradation oxydatives.

3. Utilisation selon la revendication 1 ou 2, **caractérisée en ce que** le 17α-éthinylestradiol présente une seule dose de 0,01 µg à 200 µg.

4. Utilisation selon la revendication 3, **caractérisée en ce que** le 17$\alpha$-éthinylestradiol présente une seule dose de 0,1 µg à 200 µg.

5. Utilisation selon la revendication 4, **caractérisée en ce que** le 17$\alpha$-éthinylestradiol présente une seule dose de 10 ou 20 µg.